(19) Europäisches Patentamt
European Patent Office
Office européen des brevets

(11) Veröffentlichungsnummer: **0 001 270**
**A1**

# (12) EUROPÄISCHE PATENTANMELDUNG

(21) Anmeldenummer: **78100907.1**

(22) Anmeldetag: **15.09.78**

(51) Int. Cl.²: **C 07 D 307/93**
A 61 K 31/34
//C07C177/00

(30) Priorität: **27.09.77 DE 2743283**

(43) Veröffentlichungstag der Anmeldung:
**04.04.79 Patentblatt 79/7**

(84) Benannte Vertragsstaaten:
**BE CH DE FR GB NL SE**

(71) Anmelder: **HOECHST AKTIENGESELLSCHAFT**
**Postfach 80 03 20**
**D-6230 Frankfurt/Main 80(DE)**

(72) Erfinder: **Bartman, Wilhelm, Dr.**
**Am Dachsbau 5**
**D-6232 Bad Soden am Taunus(DE)**

(72) Erfinder: **Konz, Elmar, Dr.**
**Buchenweg 22**
**D-6232 Bad Soden am Taunus(DE)**

(72) Erfinder: **Lerch, Ulrich, Dr.**
**Schwalbenweg 19**
**D-6238 Hofheim am Taunus(DE)**

(72) Erfinder: **Schölkens, Bernward, Dr.**
**Am Fliedergarten 1**
**D-6233 Kelkheim (Taunus)(DE)**

(54) **Neue Prostacyclin-Analoga, Verfahren zu ihrer Herstellung und ihre Verwendung bei der Behandlung von Herzkreislauf-Krankheiten.**

(57) Prostacyclin-Analoga der Formel

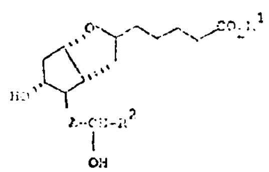

sowie Verfahren zu deren Herstellung.

Die Verbindungen zeichnen sich durch ihre relaxierende Wirkung auf die Gefäßwand, insbesondere der Coronararterien aus, ferner hemmen sie die Thrombozytenaggregation und besitzen magensaftsekretionshemmende woeie blutdrucksenkende Eigenschaften. Sie können daher als Arzneimittel, insbesondere zur Behandlung von Herzkreislaufkrankheiten verwendet werden.

EP 0 001 270 A1

Croydon Printing Company Ltd.

HOECHST AKTIENGESELLSCHAFT HOE 77/F 192 Dr.LA/Rp

# Neue Prostacyclin-Analoga, Verfahren zu ihrer Herstellung und ihre Verwendung bei der Behandlung von Herzkreislauf-Krankheiten

Prostacyclin, eine pharmakologisch hochwirksame Substanz, wurde kürzlich isoliert (Nature 263, 663, 1976) und seine Struktur aufgeklärt (Prostaglandins 12, 915, 1976). Nach neuester Übereinkunft wird Prostacyclin als $PGI_2$ bezeichnet (Prostaglandins 13, 375, 1977).

Der Naturstoff zeichnet sich aus durch eine sehr starke Hemmung der Thrombocytenaggregation (The Lancet, 1977, 18) und Relaxation einiger Blutgefäße, z.B. der Coronararterien (Prostaglandins 13, 3, 1977). Er kann zur Therapie und Prophylaxe von Thrombosen und Infarkten Verwendung finden. Wegen seiner sehr geringen Stabilität (Prostaglandins 12, 915, 1976) ist das natürliche Prostacyclin für eine breite therapeutische Anwendung jedoch wenig geeignet. Synthesen von stabileren, nicht natürlich vorkommenden Analogen von Prostacyclin sind deshalb von großem Interesse.

Die vorliegende Erfindung betrifft Prostacyclin-Analoga der allgemeinen Formel I

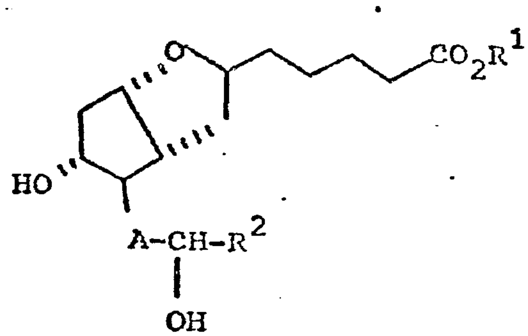

I

in welcher bedeuten

$R^1$ Wasserstoff, oder einen geradkettigen oder verzweigten Alkylrest mit 1 bis 6 Kohlenstoffatomen, oder einen geradkettigen oder verzweigten ungesättigten aliphatischen Kohlenwasserstoffrest mit 3 bis 6 Kohlenstoffatomen oder einen cycloaliphatischen Kohlenwasserstoffrest mit 3 bis 7 Kohlenstoffatomen oder einen araliphatischen Kohlenwasserstoffrest mit 7 bis 9 Kohlenstoffatomen, oder ein physiologisch verträgliches Metall-, $NH_4$- oder Ammoniumion, das sich von einem primären, sekundären oder tertiären Amin ableitet,

$R^2$ einen geradkettigen, verzweigten, gesättigten oder ungesättigten aliphatischen Kohlenwasserstoffrest mit 1 bis 8 Kohlenstoffatomen, der seinerseits substituiert sein kann durch

a) einen geradkettigen oder verzweigten Alkoxy- oder Alkenyloxyrest mit 1 bis 6 Kohlenstoffatomen oder

b) einen Cycloalkoxyrest mit 3 bis 7 Kohlenstoffatomen

A die trans 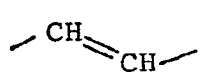 oder $-CH_2-CH_2-$Gruppe.

Gegenstand der Erfindung ist ferner ein Verfahren zur Herstellung der Verbindungen der Formel I, das dadurch gekennzeichnet ist, daß man

1 a) eine Verbindung der Formel II

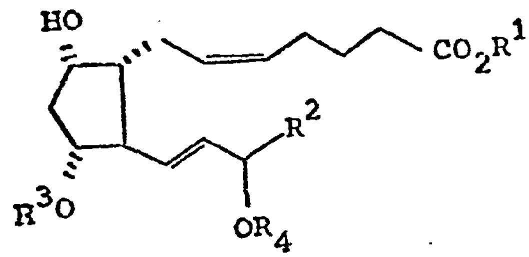

II

worin $R^1$ und $R^2$ die zur Formel I angegebene Bedeutung haben und $R^3$ und $R^4$ gleich oder verschieden sind und Wasserstoff oder eine leicht abspaltbare Schutzgruppe bedeuten, mit einem geeigneten elektrophilen Reagens zu einer Verbindung der Formel III

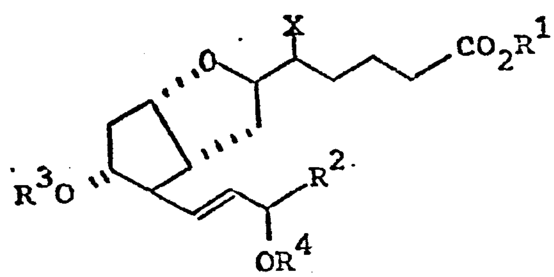

III

worin $R^1$ und $R^2$ die zur Formel I, $R^3$ und $R^4$ die zur Formel II angegebene Bedeutung haben und X ein Halogenatom, insbesondere Brom oder Jod, eine Arylselenylgruppe oder einen Rest der Formel $-Hg-O-\underset{O}{\underset{\|}{C}}-Y$ bedeutet, worin Y einen gegebenenfalls halogenierten aliphatischen Kohlenwasserstoffrest mit 1 bis 4 C-Atomen oder einen araliphatischen Kohlenwasserstoffrest mit 7 bis 9 C-Atomen darstellt,

1 $a_1$) gegebenenfalls in einer Verbindung der Formel III, worin $R^3$ und/oder $R^4$ eine Schutzgruppe bedeuten, diese abspaltet, wobei eine Verbindung der Formel III

erhalten wird, in welcher $R^1$ und $R^2$ die zur Formel I und X die zur Formel III angegebenen Bedeutungen haben und $R^3$ und $R^4$ Wasserstoff bedeuten,

1 b) aus einer Verbindung der Formel III die Gruppe X reduktiv abspaltet, wobei eine Verbindung der Formel IV

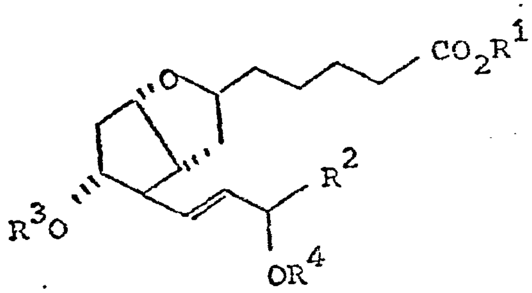

IV

erhalten wird, worin $R^1$ und $R^2$ die zur Formel I und $R^3$ und $R^4$ die zur Formel II angegebenen Bedeutungen haben,

1 c) gegebenenfalls in einer Verbindung der Formel IV, worin $R^3$ und/oder $R^4$ eine Schutzgruppe bedeuten, diese unter geeigneten Bedingungen abspaltet, wobei eine Verbindung der Formel I gebildet wird, worin A eine trans-Vinylidengruppe bedeutet und $R^1$ und $R^2$ die zur Formel I angegebenen Bedeutungen haben,

1 d) gegebenenfalls eine Verbindung der Formel I, worin $R^1$ und $R^2$ die zur Formel I angegebenen Bedeutungen haben und A eine trans-Vinylidengruppe bedeutet, zu einer Verbindung der Formel I reduziert, worin $R^1$ und $R^2$ die zur Formel I angegebenen Bedeutungen haben und A eine $-CH_2-CH_2-$Gruppe bedeutet,

1 $d_1$) gegebenenfalls eine Verbindung der Formel IV, worin $R^3$ und $R^4$ nicht gleichzeitig Wasserstoff bedeuten, zu einer Verbindung der Formel V

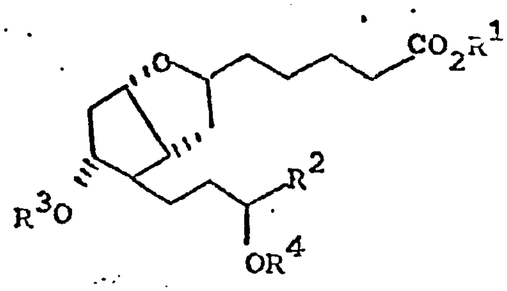

V

reduziert, worin $R^1$ und $R^2$ die zur Formel I und $R^3$ und $R^4$ die zur Formel II angegebenen Bedeutungen haben, jedoch nicht gleichzeitig Wasserstoff bedeuten, oder

1 $d_1$') eine Verbindung der Formel III direkt zu einer Verbindung der Formel V reduziert, worin $R^1$ und $R^2$ die zur Formel I und $R^3$ und $R^4$ die zur Formel II angegebenen Bedeutungen haben,

1 $d_2$) gegebenenfalls in einer Verbindung der Formel V, worin $R^3$ und/oder $R^4$ eine Schutzgruppe bedeuten, diese unter geeigneten Bedingungen abspaltet, wobei eine Verbindung der Formel I entsteht, in der $R^1$ und $R^2$ die zur Formel I angegebenen Bedeutungen haben, $R^3$ und $R^4$ Wasserstoff und A eine $CH_2$-$CH_2$-Gruppe bedeuten,

1 e) gegebenenfalls eine Verbindung der Formel I, worin $R^1$ Wasserstoff bedeutet und A und $R^2$ die zur Formel I angegebenen Bedeutungen haben, zu einer Verbindung der Formel I verestert, worin $R^1$ ein Alkylrest mit der zur Formel I angegebenen Bedeutung ist,

1 f) gegebenenfalls eine Verbindung der Formel I, worin A und $R^2$ die zur Formel I angegebenen Bedeutungen haben und $R^1$ die zur Formel I angegebene Bedeutung hat, jedoch kein Wasserstoffatom oder Kation bedeutet, umestert,

g) gegebenenfalls eine Verbindung der Formel I, worin A und $R^2$ die zur Formel I angegebenen Bedeutungen haben und $R^1$ einen Alkylrest bedeutet, zu einer Verbindung der Formel I verseift, worin $R^1$ Wasserstoff bedeutet,

h) gegebenenfalls eine Verbindung der Formel I, worin A und $R^2$ die zur Formel I angegebenen Bedeutungen haben und $R^1$ Wasserstoff bedeutet, mit Basen in eine Verbindung der Formel I überführt, worin $R^1$ ein physiologisch verträgliches Metall-, $NH_4$- oder Ammoniumion bedeutet, das sich von einem primären, sekundären oder tertiären Amin ableitet, oder

2 a) eine Verbindung der Formel VI

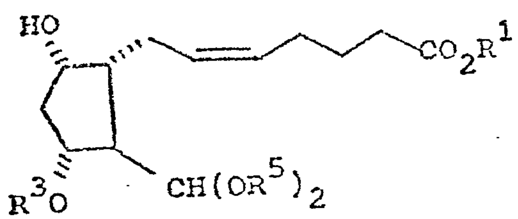

VI

worin $R^1$ die zur Formel I und $R^3$ die zur Formel II angegebenen Bedeutungen haben, und $R^5$ einen Alkylrest mit 1 bis 4 Kohlenstoffatomen bedeutet, mit einem elektrophilen Reagens umsetzt, wobei eine Verbindung der Formel VII

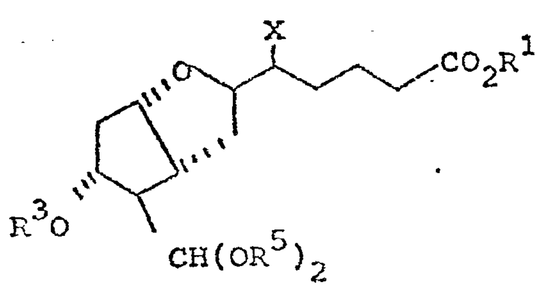

VII

gebildet wird, worin $R^1$ die zur Formel I, $R^3$ die zur Formel II, $R^5$ die zur Formel VI und X die zur Formel III angegebenen Bedeutungen haben,

2 b) aus einer Verbindung der Formel VII die Gruppe X reduktiv abspaltet, wobei eine Verbindung der Formel VIII

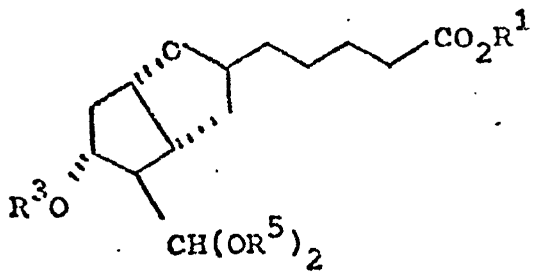

VIII

erhalten wird, worin $R^1$ die zur Formel I, $R^3$ die zur Formel II und $R^5$ die zur Formel VI angegebenen Bedeutungen haben,

2 b') gegebenenfalls in einer Verbindung der Formel VIII, worin $R^3$ Wasserstoff bedeutet, diesen durch eine Schutzgruppe ersetzt,

2 c) aus einer Verbindung der Formel VIII durch saure Hydrolyse die Acetalschutzgruppe entfernt, wobei eine Verbindung der Formel IX

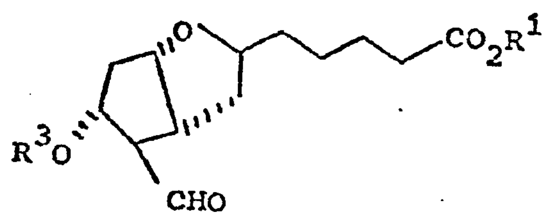

IX

entsteht, worin $R^1$ die zur Formel I und $R^3$ die zur Formel II angegebenen Bedeutungen haben,

2 d) einen Aldehyd der Formel IX mit einem Phosphonat der Formel X

$$(R^6O)_2\overset{O}{\overset{\|}{P}} - CH_2 - \overset{O}{\overset{\|}{C}} - R^2 \qquad X$$

worin $R^6$ einen Alkylrest mit 1 bis 4 Kohlenstoffatomen bedeutet und $R^2$ die zur Formel I angegebene Bedeutung hat, unter Basenkatalyse kondensiert, wobei eine Verbindung der Formel XI

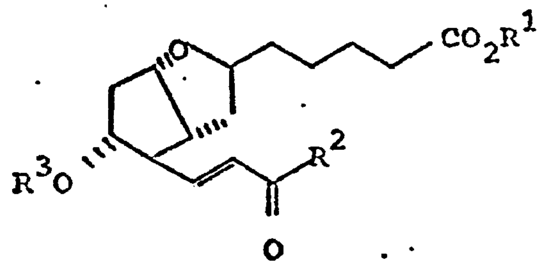

XI

entsteht, worin $R^1$ und $R^2$ die zur Formel I und $R^3$ die zur Formel II angegebene Bedeutung haben,

2 e) ein Keton der Formel XI zu einem Alkohol der Formel IV reduziert, worin $R^1$ und $R^2$ die zur Formel I, $R^3$ die zur Formel II angegebenen Bedeutungen haben und $R^4$ Wasserstoff bedeutet und gegebenenfalls weiter verfährt wie unter 1 c) bis 1 h) angegeben.

Bevorzugt sind die folgenden Reste:

Für $R^1$: Wasserstoff oder ein geradkettiger, verzweigter Alkylrest mit 1 bis 6 Kohlenstoffatomen oder ein cycloaliphatischer Kohlenwasserstoffrest mit 5 bis 7 Kohlenstoffatomen oder ein physiologisch verträgliches Metall-, $NH_4$- oder Ammonium, das sich von einem primären, sekundären oder tertiären Amin ableitet.

Für $R^2$: ein Rest der allgemeinen Formel XII

$$-(CH_2)_m-\underset{R^8}{\overset{R^7}{\overset{|}{\underset{|}{C}}}}-(CH_2)n-O-R^9 \qquad XII$$

worin bedeuten

$R^7$ und $R^8$ Wasserstoff oder eine Methyl- oder Äthylgruppe, wobei $R^7$ und $R^8$ gleich oder verschieden sein können.

$R^9$: a) einen geradkettigen oder verzweigten Alkylrest mit 1 bis 5 Kohlenstoffatomen oder

b) einen Cycloalkylrest mit 5 bis 7 Kohlenstoffatomen.

m und n: jeweils eine der Zahlen 0 - 3, wobei im Rest der allgemeinen Formel XII die Gesamtanzahl der Kohlenstoffatome zwischen 3 und 10 beträgt.

Unter den Substituenten für $R^2$ sind die im folgenden aufgeführten ganz besonders bevorzugt:

2-Oxapent-1-yl, 3-Oxahex-2-yl, 2-Methyl-3-oxahept-2-yl, 3-Äthyl-4-oxaoct-3-yl, 2-Methyl-3-oxaoct-2-yl, 3-Äthyl-4-oxahept-3-yl, 1-Methyl-1-cyclopentyloxyäth-1-yl, 2,5-Dimethyl-3-oxahex-2-yl, 3-Oxa-2,6,6-trimethylhept-2-yl, 2-Methyl-4-oxahept-2-yl, 3-Äthyl-5-oxahept-3-yl, 1-Cyclohexyloxymethyl-1-methyläthyl, 4-Oxa-2,6,6-trimethylhept-2-yl, 1-Cyclopentyloxymethyl-1-methyl-äthyl, 4-Oxapent-1-yl, 5-Oxahex-2-yl, 6-Methyl-4-oxahept-1-yl, 3-Äthyl-6-oxahept-3-yl, 2,6-Dimethyl-5-oxahept-2-yl, 2-Methyl-5-oxaoct-2-yl, 4-Cyclopentyloxy-2-methylbut-2-yl, 3,3-Dimethyl-4-oxapentyl, 5-Oxa-2,6,6-trimethylhept-2-yl, 3,3-Dimethyl-4-oxahexyl, 3,3-Dimethyl-4-oxaheptyl, 2,2-Dimethyl-4-oxahexyl, 2,2-Dimethyl-4-oxaheptyl, 2-Methyl-

6-oxahept-2-yl, 2-Methyl-6-oxaoct-2-yl, 3-Äthyl-7-oxa-oct-3-yl, 5-Oxaheptyl, 2,7-Dimethyl-6-oxaoct-2-yl, 6-Oxahept-2-yl, 2,2-DImethyl-5-oxahexyl, 2,2-Dimethyl-5-oxaheptyl, 3,3-Dimethyl-5-oxahexyl, 4,4-Dimethyl-5-oxyhexyl, 2,2-Dimethyl-6-oxaheptyl, 3,3-Dimethyl-6-oxaheptyl, 6-Oxaheptyl, 4-Methyl-6-oxaoctyl, 4,4-Dimethyl-6-oxaheptyl.

Das in dem erfindungsgemäßen Verfahren als Ausgangsmaterial verwendete Prostaglandinderivat der allgemeinen Formel II kann z.B. nach dem in JACS 91, 5675 (1969) beschriebenen Verfahren hergestellt werden.

Für die Cyclisierung von Verbindungen der Formel II zu Verbindungen der Formel III eigenen sich elektrophile Reagentien wie z.B. Quecksilbertrifluoracetat, Arylselenylhalogenide, wie z.B. Phenylselenylchlorid oder -bromid sowie Halogenierungsreagentien wie Jod, Jodchlorid, oder n-Bromimide wie N-Bromsuccinimid, N-Bromcampherimid oder 1,3-Dibrom-5,5-dimethylhydantoin. Die Reaktion wird vorzugsweise in einem inerten Lösungsmittel, wie z.B. Methylenchlorid, Chloroform, Tetrahydrofuran oder 1,2-Dimethoxyäthan durchgeführt bei Temperaturen zwischen -70 und +30°C, je nach Art des eingesetzten Reagens. Die Reaktion kann in Gegenwart eines säurebindenden Mittels wie z.B. Calciumcarbonat durchgeführt werden.

Eine bevorzugte Ausführungsform des Verfahrens besteht darin, daß man zu einer Lösung der Verbindung II THF in Gegenwart von Calciumcarbonat bei Raumtemperatur die 1- bis 1,2-molare Menge an Quecksilber-II-trifluoracetat zugibt und nach 1 bis 3 Stunden die entstandene Quecksilberverbindung III

$(X = -Hg-O-\overset{\overset{\displaystyle O}{\|}}{C}-CF_3)$ direkt dem nachfolgenden Reduktionsschritt unterwirft.

Die Entfernung der Gruppe X aus einer Verbindung der Formel III geschieht auf reduktivem Weg. Sofern X ein substituiertes Quecksilberatom bedeutet, entfernt man dieses z.B. mit einem komplexen Metallhydrid wie z.B. Natriumborhydrid, sofern X ein Jod- oder Bromatom bedeutet, läßt sich dieses z.B. mit Zinkpulver, einem Organozinnhydrid wie Tributylzinnhydrid oder einem komplexen Borhydrid wie Natriumborhydrid oder Natriumcyanoborhydrid in einem dipolaren Lösungsmittel wie DMF, DMSO, HMPT oder einem Äther wie Dimethyldiglycol (Diglyme), gegebenenfalls in Gegenwart von Wasser entfernen. Eine bevorzugte Ausführungsform des Verfahrens besteht darin, daß man die oben erhaltene Quecksilberverbindung der Formel III in situ bei etwa -30 bis +20°C, vorzugsweise bei -20°C, mit Natriumborhydrid in einem Lösungsmittel wie z.B. Äthanol reduziert, wobei eine Verbindung der Formel IV erhalten wird.

Sofern die Reste $R^3$ und/oder $R^4$ nicht Wasserstoff bedeuten, können diese abhydrolysiert werden. Handelt es sich um Acetalgruppe wie z.B. Tetrahydropyranylgruppen, so können diese unter Säurekatalyse in einem alkoholischen oder wäßrig/organischem Lösungsmittel abgespalten werden. Als Säure eignen sich sehr verdünnte Mineralsäuren oder organische Säuren wie p-Toluol-sulfonsäure, Oxalsäure oder Essigsäure. Sofern $R^3$ und/oder $R^4$ Acylgruppen bedeuten, so können diese sowohl sauer oder basisch abgespalten werden: Sauer z.B. durch Umesterung in einem alkoholischen Medium oder durch Hydrolyse in wäßriger Säure, gegebenenfalls in Gegenwart eines organischen Lösungsmittels, alkalisch in Gegenwart einer Base wie Alkalihydroxyd oder -carbonat z.B. in einem alkoholischen oder wäßrigorganischen Lösungsmittel.

Die Reduktion der Doppelbindung in Verbindungen der Formel I, worin A eine trans-Vinylidengruppe bedeutet, zu Verbindungen der Formel I, worin A eine $CH_2$-$CH_2$-Gruppe be-

deutet, kann mit allen für die Reduktion einer olefinischen Doppelbindung geeigneten Methoden erfolgen, wie z.B. mit Diimin oder mit einem Alkalimetall in einem niedermolekularen Alkohol oder Amin.

Besonders bevorzugt ist die katalytische Hydrierung mit einem geeigneten Katalysator, z.B. fein verteiltem Nickel, Palladium, Platin, gegebenenfalls auf einem Trägermaterial wie z.B. Aktivkohle, Kieselgur, Calciumcarbonat oder Bariumsulfat. Als Lösungsmittel eignen sich die für katalytische Hydrierungen gebräuchlichen, wie niedermolekulare Alkohole, Ester oder Äther, z.B. Methanol, Äthylacetat, THF oder Dimethoxyäthan.

Auf die gleiche Weise lassen sich Verbindungen der Formel IV, worin $R^3$ und/oder $R^4$ eine Schutzgruppe bedeutet, zu Verbindungen der Formel V reduzieren bzw. hydrieren.

Man kann auch in Verbindungen der Formel III die Gruppe X und die olefinische Doppelbindung in einem Schritt durch katalytische Hydrierung entfernen, insbesondere, wenn X Jod, eine Alkyl- oder Arylselenylgruppe oder ein substituiertes Quecksilberatom bedeutet.

Die Abspaltung der Schutzgruppen $R^3$ und/oder $R^4$ aus Verbindungen der Formel V erfolgt nach den gleichen Methoden, die für die Abspaltung der Schutzgruppen aus Verbindungen der Formel IV beschrieben sind. Verbindungen der Formel I ($R^1$ = H) lassen sich nach den herkömmlichen Methoden verestern. Bevorzugt ist die Veresterung der Carbonsäure mit einem Diazoalkan in einem inerten Lösungsmittel. Besonders gut eigenen sich Äther wie Diäthyläther oder THF, aber auch andere inerte Lösungsmittel wie Methylenchlorid, Methanol oder Benzol können verwendet werden.

Eine weitere bevorzugte Möglichkeit zur Herstellung dieser Ester bietet die Umsetzung von Salzen der Carbonsäuren mit

einem Alkylhalogenid. Als Lösungsmittel eigenen sich hierfür insbesondere dipolare, aprotische Lösungsmittel wie Acetonitril, Dimethylformamid oder Dimethylsulfoxyd. Die Reaktionstemperaturen können zwischen -10° und +100°C liegen, bevorzugt werden Temperaturen zwischen +20° und +60°C. Ester der Formel I lassen sich auch durch Umesterung ineinander umwandeln. Beispielsweise ist es vorteilhaft, Ester, die sich von sekundären Alkoholen oder schwer zugänglichen Diazoverbindungen ableiten, dadurch herzustellen, daß man eine Verbindung der Formel I (R = H) mit Diazomethan zum Methylester ($R^1$ = $CH_3$) umsetzt und diesen anschließend mit einem Alkalimetallalkoholat in dem entsprechenden Alkohol umestert. Das Reaktionsprodukt kann nach Neutralisieren mit Eisessig und Entfernen des überschüssigen Alkohols chromatographisch gereinigt werden.

Ester der Formel I lassen sich nach an sich bekannten Methoden sowohl säurekatalysiert als auch basenkatalysiert verseifen, z.B. im wäßrigem THF oder Dioxan in Gegenwart einer Säure wie p-Toluolsulfonsäure oder Oxalsäure. Bevorzugt sind alkalische Verseifungsmethoden, z.B. mit NaOH oder KOH in einem niedermolekularen Alkohol oder Äther wie Methanol, THF oder Dimethoxyäthan, gegebenenfalls in Gegenwart von Wasser.

Die Säuren der Formel I (R = H) lassen sich in einfacher Weise in Salze überführen, indem man die Carbonsäure mit der äquivalenten Menge einer Base in einem geeigneten Lösunsmittel, z.B. einem niedermolekularen Alkohol umsetzt und das Lösungsmittel entfernt.

Ein zweites Verfahren zur Herstellung der erfindungsgemäßen Verbindungen der Formel I besteht darin, daß man zunächst Acetale der Formel VI zu Verbindungen der Formel VII cyclisiert. Diese Cyclisierungsreaktion erfolgt nach den gleichen Methoden, wie sie für die Herstellung der Verbindungen der Formel III aus Verbindungen der Formel II beschrieben wurden.

Die Abspaltung der Gruppe X aus Verbindungen der Formel VII zu Verbindungen der Formel VIII wird nach den oben beschriebenen Methoden für die Umwandlung von Verbindungen der Formel III in Verbindungen der Formel IV durchgeführt.

Falls $R^3$ in Formel VIII Wasserstoff bedeutet, wird dieser zweckmäßigerweise durch eine geeignete Schutzgruppe ersetzt.

Als geeignete Schutzgruppen kommen in Frage: z.B. die THP-Schutzgruppen, die 1-Äthoxyäthyl- oder die 1-Methoxy-1-methyläthylgruppe, die durch säurekatalysierte Addition von 2,3-Dihydropyran, Äthylvinyläther oder Methylisopropenyläther an die Hydroxyl-Funktion erhalten werden können. Besonders geeignete Schutzgruppen sind auch Acylreste wie der Acetyl, Benzoyl- oder p-Phenylbenzoylrest. Verbindungen der Formel VIII ($R^3$ = H) können nach herkömmlichen Methoden acyliert werden, z.B. mit dem entsprechenden Säureanhydrid oder Säurechlorid in Gegenwart einer Base wie z.B. Pyridin, gegebenenfalls in einem inerten Lösungsmittel wie z.B. Dimethoxyäthan oder Methylenchlorid.

Die Freisetzung der Aldehyde IX aus den Acetalen der Formel VIII erfolgt durch saure Hydrolyse z.B. in wäßrigorganischer Phase. Als organisches Lösungsmittel eignen sich die mit Wasser mischbaren wie z.B. THF, Dioxan, 1,2-Dimethoxyäthan, Aceton oder niedermolekulare Alkchole. Man kann aber auch in einem Zweiphasensystem arbeiten, d.h. in einem Gemisch aus wäßriger Phase und einem mit Wasser mischbaren Lösungsmittel wie z.B. Chloroform, gegebenenfalls unter Zusatz eines Lösungsvermittlers.

Als Säuren kommen in Betracht Mineralsäuren wie Salzsäure, Schwefelsäure oder Phosphorsäure oder organische Säuren wie Oxalsäure oder p-Toluolsulfonsäure.

Eine bevorzugte Ausführungsform des Verfahrens besteht darin, daß man eine Lösung des Acetals VIII in Chloroform mit wäßriger Salzsäure und einer geringen Menge Isopropanol behandelt, gegebenenfalls unter Rühren. Der entstandene Aldehyd der Formel IX kann durch Eindampfen der organischen Phase isoliert werden.

Aldehyde der Formel IX können ohne weitere Reinigung für den nächsten Verfahrensschritt eingesetzt werden. Falls erforderlich können sie durch Säulenchromatographie gereinigt werden.

Die Umsetzung der Phosphonate der Formel X mit Verbindungen der Formel IX kann unter den für die Horner-Reaktion gebräuchlichen Bedingungen durchgeführt werden, beispielsweise in Äthern bei Raumtemperatur. Als Äther kommen bevorzugt in Betracht Diäthyläther, Tetrahydrofuran und Dimethoxyäthan. Das Phosphonat kann im Überschuß eingesetzt werden.

Die Reaktion ist gewöhnlich nach 3 bis 24 Stunden bei Temperaturen zwischen 10 und 50°C beendet. Das Reaktionsprodukt der Formel XI wird dann durch übliche Verfahren aus der Reaktionsmischung isoliert und gereinigt. Einzelheiten über die Durchführung dieser Reaktion sind in J. Amer. Chem. Soc. 83, 1733 (1961) beschrieben.

Die Phosphonate der Formel X sind entweder bekannt (J. Org. Chem. 30 680 (1965)) oder können analog zu bekannten Verfahren hergestellt werden (z.B. J. Amer. Chem. Soc. 88 5654 (1966)).

Verbindungen der Formel IV ($R^4$ = H) können durch die Behandlung der Verbindungen der Formel XI mit einem Reduktionsmittel erhalten werden. Die Reduktion kann mit allen Reduktionsmitteln bewerkstelligt werden, die eine selektive Reduktion einer Ketogruppe zu einer Hydroxylgruppe in Gegenwart einer olefinischen Doppelbindung ermöglichen. Bevorzugte Reduktionsmittel sind komplexe Metallhydride, insbesondere die Borhydride wie Kalium- oder Natriumborhydrid, Zinkborhydrid oder Lithiumperhydro-9b-bora-phenalylhydrid (J. Amer. Chem. Soc. 92, 709 (1970)) oder auch Aluminiumhydride wie z.B. Natrium-bis-(2-methoxy-äthoxy)-aluminiumhydrid oder Diisobutylaluminiumhydrid. Gewöhnlich wird die Reduktion zwischen -10° und +50°C in einem gegenüber den Hydriden inerten Lösungsmittel wie Äthern, z.B. Äthyläther, Dimethoxyäthan, Dioxan, Tetrahydrofuran oder Diäthylenglykoldimethyläther oder Kohlenwasserstoffen wie z.B. Benzol durchgeführt. Im Fall von Natriumborhydrid kann man auch in einem Alkohol/Wassergemisch wie z.B. Methanol/Wasser arbeiten. Eine weitere Methode zur Reduktion der Ketogruppe ist die sogenannte Meerwein-Ponndorf-Reduktion. Sie besteht darin, daß man die Carbonylverbindung der Formel XI in einem inerten Lösungsmittel wie z.B. Benzol- oder Toluol mit Aluminiumisopropylat behandelt. Das sich bildende Aceton wird aus dem Reaktionsgemisch abdestilliert. Die Reduktion der Ketofunktion in 15-Stellung (Prostaglandinnomenklatur) verläuft gewöhnlich nicht stereospezifisch. Es entsteht ein Gemisch von α- und ß-Isomeren bezüglich der Stellung der resultierenden Hydroxylgruppe in den Verbindungen der Formel IV. Ein Isomerengemisch bezüglich der Stereochemie am Kohlenstoffatom 6 (Prostaglandinnomenklatur) entsteht auch bei den Cyclisierungsreaktionen von Verbindungen der Formel II zu Verbindungen der Formel III bzw. VI ⟶ VIII.

Die entsprechende Stereoisomeren können direkt nach deren Entstehung oder auch nach jeder folgenden Reaktionsstufe getrennt werden. Das bedeutet, daß alle beschriebenen Reaktionen mit dem reinen α- bzw. ß-Isomeren durchgeführt werden können, aber auch mit α,ß-Isomerengemischen.

Die erfindungsgemäßen Verbindungen der Formel I werden gewöhnlich in Form der Racemate erhalten. Diese können gegebenenfalls nach den üblichen Methoden der Racemattrennung in Form der optisch aktiven Antipoden erhalten werden. Zweckmäßigerweise wird eine Racemattrennung nicht erst beim Endprodukt, sondern bei einem geeigneten Zwischenprodukt durchgeführt (siehe dazu z.B. JACS 92, 397 (1970)).

Sofern die einzelnen Reaktionsprodukte nicht bereits in genügend reiner Form anfallen, so daß sie für den folgenden Reaktionsschritt eingesetzt werden können, empfiehlt sich eine Reinigung mittels z.B. Säulen-, Dünnschicht- oder auch Hochdruckflüssigkeitschromatographie.

Nach den erfindungsgemäßen Verfahren lassen sich außer den in den Beispielen genannten insbesondere auch die folgenden Verbindungen herstellen:

17-Oxa-$PGI_1$-methylester
16,16-Dimethyl-17-oxa-21-homo-$PGI_1$
13,14-Dihydro-17-oxa-16,16,19-trimethyl-$PGI_1$-n-butylester
1,6,16-Dimethyl-18-oxa-21-homo-$PGI_1$
13,14-Dihydro-16,16-dimethyl-18-oxa-$PGI_1$-propylester
17-Cyclohexyloxy-16,16-dimethyl-18,19,20-trinor-$PGI_1$-methylester
13,14-Dihydro-18-oxa-16,16,20,20,20-pentamethyl-$PGI_1$
19-Oxa-$PGI_1$-äthylester
16,16-Diäthyl-19-oxa-$PGI_1$-methylester
13,14-Dihydro-19-oxa-16,16,20,20-tetramethyl-$PGI_1$-isopropylester
18,18-Dimethyl-19-oxa-$PGI_1$
16,16-Dimethyl-20-oxa-21-homo-$PGI_1$-methylester
16,16-Diäthyl-13,14-dihydro-20-oxa-21,22-dihomo-$PGI_1$
16-Methyl-20-oxa-21-homo-$PGI_1$-äthylester
17,17-Dimethyl-20-oxa-21,22-dihomo-$PGI_1$-methylester
13,14-Dihydro-18,18-dimethyl-20-oxa-21-homo-$PGI_1$

Die erfindungsgemäßen Verbindungen zeichnen sich durch ihre relaxierende Wirkung auf die Gefäßwand besonders der Coronararterien aus. Außerdem hemmen die beanspruchten Verbindungen die Thrombocytenaggregation, und besitzen magensaftsekretionshemmende und blutdrucksenkende Eigenschaften, wie durch in vitro-Tests bzw. in vivo-Tests z.B. am Hund ermittelt wurde. Sie können daher als Arzneimittel angewandt werden.

Als Dosiseinheit kommen 0,1 bis 50 mg, als Tagesdosis 0,5 bis 100 mg in Betracht.

Die erfindungsgemäßen Verbindungen der Formel I können als freie Säuren, in Form ihrer physiologisch unbedenklichen anorganischen oder organischen Salze oder als Ester zu Anwendung kommen.

Säure und Salze bzw. Ester können in Form ihrer wäßrigen Lösungen oder Suspensionen oder auch gelöst oder suspendiert in pharmakologisch unbedenklichen organischen Lösungsmitteln wie ein- oder mehrwertigen Alkoholen wie z.B. Äthanol, Äthylenglykol oder Glycerin, Ölen wie z.B. Sonnenblumenöl oder Lebertran, Äthern wie z.B. Diäthylenglykoldimethyläther oder auch Polyäthern wie z.B. Polyäthylenglykol oder auch in Gegenwart anderer pharmakologisch unbedenklicher Polymerträger wie z.B. Polyvinylpyrrolidon zur Anwendung gelangen.

Als Zubereitungen kommen die üblichen galenischen Infusions- oder Injektionslösungen sowie Tabletten und Suppositorien infrage.

Eine weitere Anwendung der neuen Verbindungen liegt in der Kombination mit anderen Wirkstoffen. Neben anderen geeigneten Substanzen gehören vor allem:

Kreislaufmittel im weitesten Sinne, wie z.B. Herzglycoside

wie Digitoxin, Sympathomimetica wie Suprifen, ß-Sympatholytica wie Inderal, Coronardilatatoren wie Chromonar oder Prenylamin, blutdrucksenkende Stoffe wie Reserpin oder Clonidin, Antiarrhythmica, durchblutungsfördernde Stoffe, Antikoagulantien oder Fibrinolytica, Diuretica, wie z.B. Furosemid, Lipidsenker oder Geriatrika und andere stoffwechselwirksamen Präparate, Prostaglandine oder Prostaglandinantagonisten oder Prostaglandin-Biosynthesehemmer, wie z.B. nichtsteroidale Antiphlogistika, Thromboxan-Synthetasehemmer, Psychopharmaka sowie Vitamine.

Die Verbindungen der Formel III, IV, V, VII, VIII, IX und XI sind neue wertvolle Zwischenprodukte für die Herstellung von Verbindungen der Formel I.

Beispiel 1:

a) 16,16-Dimethyl-17-oxa-$PGI_1$ (I)

In 10 ml wasserfreiem Tetrahydrofuran werden 512 mg Quecksilber (II) trifluoracetat und 150 mg Calciumcarbonat vorgelegt. Unter Argon und Rühren tropft man bei 0° 547 mg 16,16-dimethyl-17-oxa-$PGF_{2\alpha}$-11,15-bis-tetrahydropyranyläther in 10 ml wasserfreiem Tetrahydrofuran zu. Danach läßt man eine Stunde bei Raumtemperatur nachrühren. Die Dünnschichtchromatographie an Silicagelplatten mit Cyclohexan/Äthylacetat/Eisessig 20 : 80 : 1 zeigt, daß sich das Ausgangsmaterial quantitativ umgesetzt hat (das Quecksilberderivat bleibt in der Nähe des Ursprungs). Das metallorganische Zwischenprodukt (Formel III, X = $Hg - O - \overset{O}{\overset{\|}{C}} - CF_3$) wird direkt weiter umgesetzt:

In die auf -20° gekühlte Reaktionslösung wird eine Lösung von 181,5 mg Natriumborhydrid in 10 ml wasserfreiem Äthanol zugetropft. Man läßt eine Stunde weiterrühren und filtriert dann die Reaktion durch ein Klärschichtfilter. Man gießt auf eine Mischung von Eis und Kochsalz, bringt die wäßrige Phase auf pH 3 - 4 und extrahiert das Produkt mit Chloroform. Die vereinigten organischen Phasen werden mit Wasser gewaschen,mit wasserfreiem Magnesiumsulfat getrocknet. Der rohe Bis-tetrahydro-pyranyläther (Formel IV, $R^3 = R^4$ = THP) wird direkt weiterumgesetzt:

580 mg des rohen Bis-tetrahydropyranyläthers werden in 5 ml Eisessig/Tetrahydrofuran/Wasser 3:1:1 6 Stunden bei 45° unter Argon gerührt. Das Lösungsmittel wird anschließend im Vakuum abgedampft und das ölige Rohprodukt an 35 g Silicagel mit Cyclohexan/Äthylacetat/Eisessig 30:70:1 (200 ml), 10:90:1 (250 ml), danach Äthylacetat/Eisessig 98:2 chromatographiert.

Man erhält zwei Isomere, die sich in der Konfiguration am Kohlenstoffatom 6 unterscheiden.

NMR δ 5,5 - 5,8 (m 2 H) olefin. Protonen,
3,6 - 4,5 (m, 4 ) CHOH, überlagert von (s,3) O<u>H</u>
3,3 (t, 2H) $OCH_2$,
1,1 ( 6H) $C(CH_3)_2$
0.9 (t, 3H) $CH_2-\underline{CH_3}$

b) <u>16-Methyl-17-oxa-ω-homo-PGI$_1$</u>

Reaktion analog Beispiel 1a aus 16-Methyl-17-oxa-21-homo-$PGF_{2\alpha}$-11,15-bis-tetrahydropyranyläther

NMR δ 3,6 - 4,5 (m, 5H)
1,1 (d, 3H) $CH-\underline{CH_3}$
0,9 (t, 3H) $CH_2-\underline{CH_3}$

c) <u>17-Oxa-ω-dihomo-PGI$_1$</u>

Reaktion analog Beispiel 1 a aus 17-Oxa-21,22-dihomo-$PGF_{2\alpha}$-11,15-bis-tetrahydropyranyläther

NMR δ 5,4 - 5,7 (m, 2H) olefinische Protonen,
4,6 (breites s, 3H) OH,
3,2 - 4,3 (m, 8H) $OCH_2$ und OC<u>H</u>,
0,9 (t, 3H) $CH_2-\underline{CH_3}$

d) <u>16,16-Dimethyl-16-cyclohexyloxy-17,18,19,20-tetranor-PGI$_1$</u>

Reaktion analog Beispiel 1a aus 16,16-Dimethyl-16-cyclohexyloxy-17,18,19,20-tetranor-$PGF_{2\alpha}$-11,15-bis-tetrahydropyranyläther

NMR δ 3,3 - 4,3 (m, 5H) C<u>H</u>-O,
1,2 ( 6H) $C(CH_3)_2$,
5,4 - 5,7 (m, 2H) olef. Protonen

e) 16,16-Dimethyl-18-oxa-$PGI_1$

Reaktion analog Beispiel 1 a aus 16,16-Dimethyl-18-oxa-$PGF_{2\alpha}$-11,15-bis-tetrahydropyranyläther

NMR $\delta$ 5,5 - 5,8 (m, 2H) olefinische Protonen,
4,9 (breites s, 3H) OH,
3,3 (s, 2H) $O-\underline{CH_2}-C(CH_3)_2$,
3,45 (q, 2H) $O-\overline{CH_2}-CH_3$

f) 16,16-Dimethyl-18-oxa-$\omega$-dihomo-$PGI_1$

Reaktion analog Beispiel 1 a aus 16,16-Dimethyl-18-oxa-$\omega$-dihomo-$PGF_{2\alpha}$-11,15-bis-tetrahydro-pyranyläther

NMR: $\delta$ 0,9 (s, 6H) $C(CH_3)_2$ und (t, 3H) $CH_2-\underline{CH_3}$
3,3 (s, 2H) $CH_2O$

g) 16,16-Diäthyl-18-oxa-$PGI_1$

Reaktion analog Beispiel 1 a aus 16,16-Diäthyl-18-oxa-$PGF_{2\alpha}$-11,15-bis-tetrahydropyranyläther

NMR $\delta$ 5,4 - 5,7 (m, 2H) olefinische Protonen,
4,6 (breites s, 3H) OH,
3,4 (q, 2H) $O-\underline{CH_2}-CH_3$,
1,15 (t, 3H) $O-\overline{CH_2}-CH_3$,
0,9 (t, 6H) $C(CH_2-\underline{\overline{CH_3}})_2$

h) 16,16-Dimethyl-19-oxa-$PGI_1$

Reaktion analog Beispiel 1a aus 16,16-Dimethyl-19-oxa-$PGF_{2\alpha}$-11,15-bis-tetrahydropyranyläther

NMR 5,5 - 5,7 (m, 2H) olefinische Protonen,
4,7 (breites s, 3H) OH
3,5 - 4,3 (m 4H) CH-O,

3,5 (t, 2H) $OCH_2$
3,4 (s, 3H) $OCH_3$
0,95( 6H) $C(CH_3)_2$

i) <u>17,17-Dimethyl-19-oxa-PGI$_1$</u>

Reaktion analog Beispiel 1 a aus 17,17-Dimethyl-19-oxa-PGF$_{2\alpha}$-11,15-bis-tetrahydropyranyläther

NMR$\delta$ 3,4 (s, 2H) $OCH_2$,
3,3 (s, 3H) $OCH_3$
0,9 (s, 6H) $C(CH_3)_2$

j) <u>20- Oxa-$\omega$-dihomo-PGI$_1$</u>

Reaktion analog Beispiel 1 a aus 20-Oxa-$\omega$-dihomo-PGF$_2$ - 11,15-bis-tetrahydropyranyläther

NMR$\delta$ 3,4 - 3,7 (t und q,4H) $OCH_2$,
1,15(t, 3H) $CH_2$-$CH_3$

<u>Beispiel 2:</u>

<u>16,16-Dimethyl-19-oxa-PGI$_1$</u>

Zu 100 ml abs. Tetrahydrofuran werden 2,2 g 16,16-Dimethyl-19-oxa-PGF$_{2\alpha}$-11,15-bis-tetrahydropyranyläther und 0,8 g Calciumcarbonat gegeben und die Mischung unter einer Argonatmosphäre auf -70° gekühlt.Unter Rühren tropft man 0,92 g Phenylselenylchlorid in 50 ml absolutem Tetrahydrofuran zu dieser Mischung und läßt nach Beendigung der Zugabe die Temperatur innerhalb von zwei Stunden auf -25° ansteigen. Die Suspension wird danach durch ein Klärschichtfilter gesaugt und das Filtrat bei Raumtemperatur mit etwa 50 g Raney-Nickel (hergestellt nach derin L.F. Fieser und M. Fieser, "Reagents for Organic Synthesis" Vol. <u>1</u>, Wiley and Sons, Inc. New York, N.Y. 1967 auf Seite 729

angegebenen Methode) unter einer Argonatomosphäre gerührt. Nach dem Abtrennen des Raney- Nickels und Entfernen des Lösungsmittels hinterbleibt der ölige 16,16-Dimethyl-19-oxa-$PGI_1$-11,15-bis-tetrahydropyranyläther. Zur Abspaltung der Schutzgruppen wird das Rohprodukt in einem Gemisch von Eisessig/Tetrahydrofuran/Wasser 3:1:1 etwa 6 Stunden auf 40 - 45° erwärmt. Danach wird das Lösungsmittel im Vakuum entfernt und der Rückstand an 80 g Silicagel chromatographiert. Als Laufmittel werden verwendet Äthylacetat/Eisessig 99:1 (250 ml)und 97:3 (500 ml).

NMR $\delta$ 3,5 - 4,3 (m, 4H) CH-O,
3,5 (t, 2H) $OCH_2$
3,4 (s, 3H) $OCH_3$

Beispiel 3:

16,16-Dimethyl-18-oxa-$PGI_1$

1,1 g 16,16-Dimethyl-18-oxa-$PGF_{2\alpha}$-11,15-bistetrahydropyranyläther werden in 30 ml Tetrahydrofuran/Chloroform 1:1 unter Argon und Rühren bei Raumtemperatur mit 395 mg N-Bromsuccinimid behandelt. Nach zwei Stunden wurde das Lösungsmittel im Vakuum entfernt, und der Rückstand in 15 ml Eisessig/Wasser/Tetrahydrofuran 3:1:1 unter Argon 5 Stunden bei 45° gerührt. Das Lösungsmittel wird im Vakuum entfernt, der Rückstand durch Filtrieren über eine mit 40 g Silicagel gefüllte Säule gereinigt. Als Elutionsmittel dient Benzol/Dioxan/Eisessig 20:15:1. Das nach dem Eindampfen erhaltene ölige Rohprodukt wird in 10 ml abs. Benzol gelöst und unter Argon 3 Stunden bei 60° mit 750 mg Tri-n-butylzinnhydrid und 20 mg Azoisobuttersäurenitril gerührt. Nach dem Erkalten wird die Lösung auf eine Chromatographiesäule mit 50 mg Silicagel gegeben und das Produkt mit Essigester/Eisessig 97:3 eluiert.

NMR $\delta$ 5,5 - 5,8 (m, 2H) olefin. Protonen
3,3 (s, 2H) $O-CH_2-C(CH_3)_2$,
3,45 (q, 2H) $O\overline{CH_2}-CH_3$,
1,1 (t, 3H) $O-\overline{CH_2}-CH_3$

Beispiel 4:

16,16-Dimethyl-17-oxa-$PGI_1$-methylester

40,7 mg 16,16-Dimethyl-17-oxa-$PGF_2$-methylester werden in 1 ml $H_2O$ unter Argon mit 50,8 mg Jod, 33 mg Kaliumjodid und 21,2 mg Natriumcarbonat zwei Stunden bei 5 - 10°C gerührt. Durch Zugabe von wäßriger Natriumthiosulfatlösung wird das überschüssige Jod entfärbt und das Reaktionsgemisch anschließend dreimal mit Chloroform extrahiert. Die organische Phase wird anschließend mit Wasser gewaschen, getrocknet und eingedampft.

Das ölige Rohprodukt ist chromatographisch sauber. Es wird in 1,5 ml abs. Benzol gelöst und unter Argon mit 40 mg Tributylzinnhydrid und 2 mg Azoisobuttersäurenitril 3 Stunden auf 60°C erhitzt. Nach dem Erhalten wird die Reaktionsmischung an Silicagel (etwa 2 g) gereinigt (Elutionsmittel: Äthylacetat).
NMR: 5,5 - 5,75 (2H, m) CH=CH, 3,5 - 4,4
(4H, m) C$\underline{H}$-O, überlagert von 3,65 (s, 3 H)
3,3 (2H, t) $CH_2-O$

Beispiel 5:

a) 16,16-Dimethyl-17-oxa-13,14-dihydro-$PGI_1$

270 mg 16,16-Dimethyl-17-oxa-$PGI_1$ aus Beispiel 1 a wird in der Schüttelente in 20 ml Äthanol gelöst, mit 5 %igem Palladium auf Kohle hydriert. Nach 3 - 5 Stunden ist die Wasserstoffaufnahme beendet. Der

Katalysator wird abgetrennt, mit Äthanol gewaschen und das Filtrat im Vakuum eingedampft.

NMR kein Signal zwischen $\delta$ 5 - 6
$\delta$ 3,3 (t, 2H) $OCH_2$,
1,1 ( 6H) $C(CH_3)_2$,
0,9 (t, 3H) $CH_2$-$\underline{CH_3}$

b) <u>16,16-Dimethyl-18-oxa-13,14-dihydro-PGI$_1$</u>

Reaktion analog Beispiel 5 a aus 16,16-Dimethyl-18-oxa-$PGI_1$

NMR kein Signal zwischen $\delta$ 5 - 6
$\delta$ 3,3 (s, 2H) O-$CH_2$-$C(CH_3)_2$,
4,7 (breites s, 3H) OH,
3,4 (q, 2H) O-$\underline{CH_2}$-$CH_3$

<u>Beispiel 6:</u>

<u>17,17-Dimethyl-19-oxa-13,14-dihydro-PGI$_1$</u>

Aus 17,17-Dimethyl-19-oxa-$PGF_{2\alpha}$-11,15-bis-tetrahydropyranyläther wird, wie in Beispiel 1 a beschrieben, der 17,17-Dimethyl-19-oxa-$PGI_1$-11,15-bis-tetrahydropyranyläther hergestellt. Dieser wird in roher Form wie in Beispiel 5a beschrieben mit Palladium/Kohle in Tetrahydrofuran hydriert. Der Katalysator wird abfiltriert, das Filtrat im Vakuum konzentriert und der Rückstand, wie in Beispiel 1a beschrieben, mit einer Mischung von Eisessig/Tetrahydrofuran/Wasser 3:1:1 bei 45° behandelt. Nach etwa 6 Stunden sind die Schutzgruppen abgespalten. Das Lösungsmittel wird im Vakuum entfernt und der Rückstand an Silicagel chromatographiert, wobei die in Beispiel 1a beschriebenen Elutionsmittel verwendet werden.

NMR kein Signal zwischen $\delta$ 5 - 6
$\delta$ 3,4 (s, 2H) $OCH_2$,
3,3 (s, 3H) $OCH_3$,
0,9 (s, 6H) $C(CH_3)_2$

Beispiel 7:

a) 16,16-Dimethyl-18-oxa-$PGI_1$-methylester

Eine Lösung von 385 mg (1 mMol) 16,16-Dimethyl-18-oxa-$PGI_1$ in 5 ml abs. Äther wird mit 2 ml einer 0,7 M Lösung von Diazomethan in Äther versetzt. Nach 30 minütigem Rühren bei Raumtemperatur wird das Lösungsmittel im Vakuum verdampft. Man erhält den öligen Methylester, der chromatographisch einheitlich ist.

NMR 5,5 - 5,7 (m, 2H) olefinische Protonen,
3,65 (s, 3H) $CO_2CH_2$,
3,3 (s, 2H) $O-CH_2-C(CH_3)_2$
3,4 (q, 2H) $O-CH_2-CH_3$,
1,15(t, 3H) $O-CH_2-CH_3$

b) 16,16-Dimethyl-19-oxa-$PGI_1$-n-hexylester

Zu einer Lösung von 154 mg (0,4 mMol) 16,16-Dimethyl-19-oxa-$PGI_1$ in 2 ml abs. DMF gibt man unter Rühren und unter Argonatmosphäre 33 mg (0,6 mMol) Natriummethylat. Nach 5 Minuten läßt man 127 mg (0,6 mMol) 1-Jodhexan folgen und erwärmt auf 50°. Nach 3 Stunden wird das Reaktionsgemisch zwischen Toluol und Wasser verteilt, die organische Phase getrocknet und eingedampft. Der Rückstand wird an 7 g $SiO_2$ chromatographiert. Als Elutionsmittel wird Tetrachlorkohlenstoff/Aceton 80:30 (100 ml) danach 70:30 benutzt.

NMR 3,5 - 4,3 (m, 4H) CH-O, überlagert von
4,0 (t, 2H) $-CO_2-CH_2-$,
3,5 (t, 2H) $OCH_2$
3,4 (s, 3H) $OCH_3$

c) 17-Oxa-ω-dihomo-$PGI_1$-cyclohexylester

Aus 193 mg (0,5 mMol) 17-Oxa-ω-dihomo-$PGI_1$ wird analog Beispiel 7a mit einer ätherischen Diazomethanlösung der 17-Oxa-ω-dihomo-$PGI_1$-methylester hergestellt. Dieser wird in roher Form mit einer Lösung von 100 mg Natrium in 2 ml Cyclohexanol versetzt und unter Rühren drei Stunden auf 35° erwärmt. Danach wird mit Eisessig neutralisiert und zwischen Toluol und Wasser verteilt. Die organische Phase wird mit Magnesiumsulfat getrocknet, im Vakuum vom Lösungsmittel befreit und der Rückstand an 10 g Silicagel chromatographiert (Cyclohexan/Äthylacetat/Eisessig 50:50:1).

NMR δ 5,5 - 5,7 (m, 2H) olefinische Protonen,
4,7 (breites m, 1H) $CO_2$-C<u>H</u>,
3,2 - 4,3 (m, 8H) $OCH_2$ und OCH

Beispiel 8:

a) 5-(6-exo-Dimethoxymethyl-7-endo-hydroxy-2-oxabicyclo-/3,3,0/oct-3-yl)-valeriansäuremethylester

6,04 g (20 mMol) 7-(2α,4α-Dihydroxy-5ß-dimethoxymethylcyclopent-1α-yl)-(Z)-5-heptensäure in 150 ml trockenem Tetrahydrofuran tropft man bei -10° unter Rühren und Argon zu einer Suspension von 10,25 g Quecksilbertrifluoracetat und 3,0 g Calciumcarbonat in 150 ml trockenem Tetrahydrofuran. Nach der Zugabe läßt man auf Raumtemperatur kommen und eine weitere Stunde nachrühren. Danach kühlt man das Reaktionsgemisch auf -20° und dampft eine Lösung von 3,63 g

Natriumborhydrid in 150 ml Äthanol unter Rühren hinzu. Man läßt eine Stunde bei -10° weiterrühren, filtriert die Reaktionsmischung durch ein Klärschichtfilter und befreit das Filtrat im Vakuum vom Lösungsmittel. Der Rückstand wird zwischen Wasser und Chloroform verteilt, wobei die wäßrige Phase mit Natriumdihydrogenphosphat-Lösung auf pH 3 - 4 gestellt wird. Die Chloroformphase wird getrocknet und mit einer ätherischen Diazomethanlösung behandelt (etwa 50 ml einer 0,6 M Lösung). Nach einer halben Stunde wird das Lösungsmittel im Vakuum entfernt und der Rückstand an 250 g Silicagel chromatographiert.

NMR 4,25 (d, 1H) 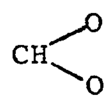

3,5 - 4,2 (m, 3H) CH-O, überlagert von

3,6 (s, 3H) $\overset{O}{\overset{\|}{C}}-OCH_3$

3,4 (s, 6H) $OCH_3$

b) <u>5-(6-exo-Dimethoxymethyl-7-endop-phenyl-benzoyloxy-2-oxabicyclo/3,3,0/oct-3-yl)-valeriansäuremethylester</u>

4,74 g 5-(6-exo-Dimethoxymethyl-7-endo-hydroxy-2-oxabecyclo/3,3,0/oct-3-yl)-valeriansäuremethylester werden in 30 ml absolutem Pyridin gelöst und unter Argon, Rühren und Kühlung auf 0° portionsweise mit 3,56 g 4-Phenylbenzoylchlorid versetzt. Nach zweistündigem Rühren bei Raumtemperatur wurde die Reaktionslösung auf ein Gemisch von Eis und Äther gegeben und mit 2N HCl auf pH 3 - 4 angesäuert. Die org. Phase wurde zweimal mit gesättigter Natriumhydrogencarbonatlösung gewaschen, getrocknet und im Vakuum vom Lösungsmittel befreit.

NMR 7,3 - 8,3 (m, 9H) arom. Protonen

4,6 (d, 1H) 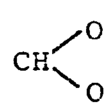

3,6 (s, 3H) $CO_2CH_3$
3,4 (d, 6H) $CH(OCH_3)_2$

c) 5-(6-exo-Formyl-7-endo-p-phenylbenzoyloxy-2-oxabicyclo /3,3,0/oct-3-yl)-valeriansäuremethylester

Eine Lösung von 4,05 5-(6-exo-Dimethoxymethyl-7-endo-p-phenylbenzoyloxy-2-oxabicyclo/3,3,0/oct-3-yl)-valeriansäuremethylester wird in 40 ml Chloroform und 1 ml Isopropanol gelöst und unter Argon und Rühren mit 4,2 ml konzentrierter Salzsäure versetzt. Nach 1 Std. bei Raumtemperatur wird 25 ml Wasser zugegeben und die organische Schicht mit gesättigter Natriumhydrogencarbonatlösung gewaschen. Nach dem Trocknen der organischen Lösung mit Magnesiumsulfat und Verdampfen des Lösungsmittels erhält man den rohen Aldehyd, der direkt weiter umgesetzt wird.

NMR 10,6 (d, 1H) CH=O
7,3 - 8,3 (m, 9H) aromat. Prot.
5,4 (m, 1H) >CH-O-CO-

d) 15-Dehydro-16,16-dimethyl-18-oxa-11-p-phenyl-benzoyl-$PGI_1$-methylester

Zu einer Suspension von 165 mg Natriumhydrid in 20 ml abs. 1,2-Dimethoxyäthan tropft man eine Lösung von 1,73 g Dimethyl-2-oxo-3,3-dimethyl-4-äthoxy-butyl-phosphonat in 10 ml trockenem 1,2-Dimethoxyäthan. Man rührt bei Raumtemperatur unter Argon eine weitere Stunde und tropft danach 2,84 g 5-(6-exo-Formyl-7-endo-p-phenylbenzoyloxy-2-oxabicyclo/3,3,0/oct-3-yl)valeriansäuremethylester in 10 ml abs. 1,2-Dimethoxyäthan zu. Nach 90 Minuten wird mit Eisessig neutralisiert, etwas Aktivkohle zugesetzt und über ein Klärschichtfilter abgesaugt. Das Filtrat wird im Vakuum konzentriert und der Rückstand an 150 g Silicagel chromatographiert. Als

Elutionsmittel dient Chlorofotm/Essigester 8:2.

NMR 7,3 - 8,2 (m, 9H) arom. Protonen
6,65- 6,85 (m, 2H) olefinische Protonen
3,4 (s, 2H und q, 2H) $O-CH_2-C(CH_3)_2$ und $O-\underline{CH_2}-CH_3$
1,12 (s, 6H) $C(CH_3)_2$

e) <u>16,16-Dimethyl-18-oxa-11-p-phenylbenzoyl-PGI$_1$-methylester</u>

Zu einer Lösung von 1,15 g 15-Dehydro-16,16-dimethyl-18-oxa-11-p-phenylbenzoyl-PGI$_1$-methylester in 20 ml abs. 1,2-Dimethoxyäthan tropft man bei 0° unter Rühren und Argon innerhalb von 10 Minuten 8 ml einer 0,5 molaren Lösung von Zinkborhydrid in 1,2-Dimethoxyäthan. Nach zwei Stunden wird durch Zugabe von gesättigter wäßriger Natriumbitartratlösung das überschüssige Borhydrid zerstört und der größte Teil des Lösungsmittels im Vakuum entfernt. Der Rückstand wird zwischen Essigester und Wasser verteilt und die organische Phase nach dem Trocknen mit Magnesiumsulfat eingedampft.

NMR 5,6 - 5,8 (m, 2H) olefinische Protonen
3,25(s, 2H) $O-CH_2-C(CH_3)_2$
0,85(d, 6H) $C(CH_3)_2$

f) <u>16,16-Dimethyl-18-oxy-PGI$_1$-methylester</u>

1,2 g des rohen 16,16-Dimethyl-18-oxa-11-p-phenylbenzoyl-PGI$_1$-methylesters werden in 20 ml absolutem Methanol gelöst und mit 400 mg pulverisiertem wasserfreiem Kaliumcarbonat versetzt. Unter Argon wird 3 Stunden gerührt und danach mit Oxalsäure auf pH 5 angesäuert. Nach dem Verdampfen des Methanols im Vakuum wird der Rückstand zwischen Essigester und gesättigter Natriumhydrogencarbonatlösung verteilt und ggf. vom

schwerlöslichen p-Phenylbenzoesäuremethylester abfiltriert. Die organische Phase wird nach dem Trocknen mit Magnesiumsulfat eingedampft und der Rückstand an 50 g Silicagel chromatographiert.

Elutionsmittel: Cyclohexan/Äthylacetat 20:80 (250 ml), danach 10:90

NMR 5,5 - 5,7 (m, 2H) olefinische Protonen
3,7 - 4,4 (m,4H) >CH-OH,
3,65 (s, 3H) $CO_2CH_3$
3,3 (s, 2H) O-$CH_2$-C$(CH_3)_2$,
1,15 (t, 3H) O-$CH_2$-$CH_3$

Patentansprüche:

1. Verbindungen der Formel I

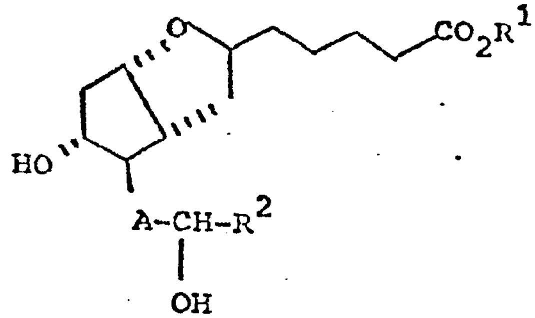

I

in welcher bedeuten

$R^1$ Wasserstoff, oder einen geradkettigen oder verzweigten Alkylrest mit 1 bis 6 Kohlenstoffatomen, oder einen geradkettigen oder verzweigten ungesättigten, aliphatischen Kohlenwasserstoffrest mit 3 bis 6 Kohlenstoffatomen oder einen cycloaliphatischen Kohlenwasserstoffrest mit 3 bis 7 Kohlenstoffatomen oder einen araliphatischen Kohlenwasserstoffrest mit 7 bis 9 Kohlenstoffatomen, oder ein physiologisch verträgliches Metall-, $NH_4$- oder Ammoniumion, das sich von einem primären, sekundären oder tertiären Amin ableitet,

$R^2$ einen geradkettigen, verzweigten, gesättigten oder ungesättigten aliphatischen Kohlenwasserstoffrest mit 1 bis 8 Kohlenstoffatomen, der seinerseits substituiert sein kann durch

a) einen geradkettigen oder verzweigten Alkoxy- oder Alkenyloxyrest mit 1 bis 6 Kohlenstoffatomen oder

b) einen Cycloalkoxyrest mit 3 bis 7 Kohlenstoffatomen

A die trans 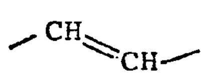 oder $-CH_2-CH_2-$Gruppe.

2. Verfahren zur Herstellung von Verbindungen der Formel I, dadurch gekennzeichnet, daß man

1 a) eine Verbindung der Formel II

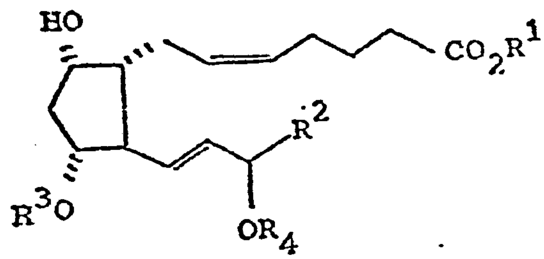

II

worin $R^1$ und $R^2$ die zur Formel I angegebene Bedeutung haben und $R^3$ und $R^4$ gleich oder verschieden sind und Wasserstoff oder eine leicht abspaltbare Schutzgruppe bedeuten, mit einem geeigneten elektrophilen Reagens zu einer Verbindung der Formel III

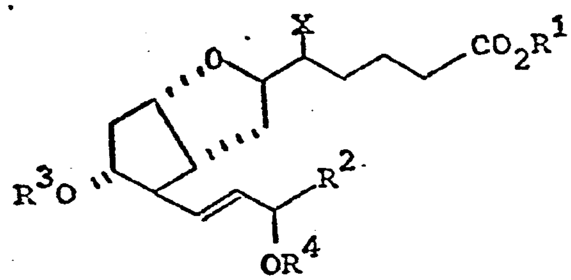

III

worin $R^1$ und $R^2$ die zur Formel I, $R^3$ und $R^4$ die zur Formel II angegebene Bedeutung haben und X ein Halogenatom, insbesondere Brom oder Jod, eine Arylselenylgruppe oder einen Rest der Formel $-Hg-O-\underset{\overset{\|}{O}}{C}-Y$ bedeutet, worin Y einen gegebenenfalls halogenierten aliphatischen Kohlenwasserstoffrest mit 1 bis 4 C-Atomen oder einen araliphatischen Kohlenwasserstoffrest mit 7 bis 9 C-Atomen darstellt,

1 $a_1$) gegebenenfalls in einer Verbindung der Formel III, worin $R^3$ und/oder $R^4$ eine Schutzgruppe bedeuten, diese abspaltet, wobei eine Verbindung der Formel III erhalten wird, in welcher $R^1$ und $R^2$ die zur Formel I und X die zur Formel III angegebenen Bedeutungen haben und $R^3$ und $R^4$ Wasserstoff bedeuten,

1 b) aus einer Verbindung der Formel III die Gruppe X reduktiv abspaltet, wobei eine Verbindung der Formel IV

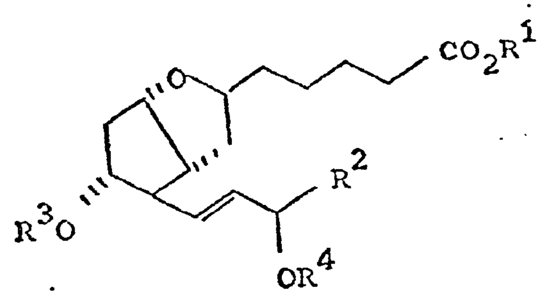

IV

erhalten wird, worin $R^1$ und $R^2$ die zur Formel I und $R^3$ und $R^4$ die zur Formel II angegebenen Bedeutungen haben,

1 c) gegebenenfalls in einer Verbindung der Formel IV, worin $R^3$ und/oder $R^4$ eine Schutzgruppe bedeuten, diese unter geeigneten Bedingungen abspaltet, wobei eine Verbindung der Formel I gebildet wird, worin A eine trans-Vinylidengruppe bedeutet und $R^1$ und $R^2$ die zur Formel I angegebenen Bedeutungen haben,

1 d) gegebenenfalls eine Verbindung der Formel I, worin $R^1$ und $R^2$ die zur Formel I angegebenen Bedeutungen haben und A eine trans-Vinylidengruppe bedeutet, zu einer Verbindung der Formel I reduziert, worin $R^1$ und $R^2$ die zur Formel I angegebenen Bedeutungen haben und A eine $-CH_2-CH_2-$Gruppe bedeutet,

1 $d_1$) gegebenenfalls eine Verbindung der Formel IV, worin $R^3$ und $R^4$ nicht gleichzeitig Wasserstoff bedeuten, zu einer Verbindung der Formel V

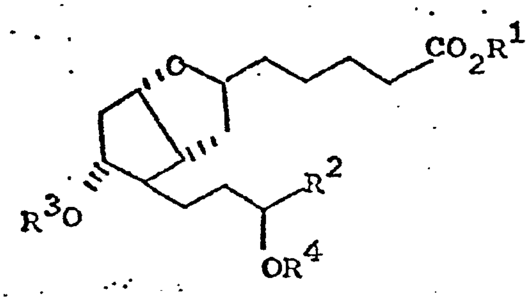

V

reduziert, worin $R^1$ und $R^2$ die zur Formel I und $R^3$ und $R^4$ die zur Formel II angegebenen Bedeutungen haben, jedoch nicht gleichzeitig Wasserstoff bedeuten, oder

1 $d_1$') eine Verbindung der Formel III direkt zu einer Verbindung der Formel V reduziert, worin $R^1$ und $R^2$ die zur Formel I und $R^3$ und $R^4$ die zur Formel II angegebenen Bedeutungen haben,

1 $d_2$) gegebenenfalls in einer Verbindung der Formel V, worin $R^3$ und/oder $R^4$ eine Schutzgruppe bedeuten, diese unter geeigneten Bedingungen abspaltet, wobei eine Verbindung der Formel I entsteht, in der $R^1$ und $R^2$ die zur Formel I angegebenen Bedeutungen haben, $R^3$ und $R^4$ Wasserstoff und A eine $CH_2$-$CH_2$-Gruppe bedeuten,

1 e) gegebenenfalls eine Verbindung der Formel I, worin $R^1$ Wasserstoff bedeutet und A und $R^2$ die zur Formel I angegebenen Bedeutungen haben, zu einer Verbindung der Formel I verestert, worin $R^1$ ein Alkylrest mit der zur Formel I angegebenen Bedeutung ist,

1 f) gegebenenfalls eine Verbindung der Formel I, worin A und $R^2$ die zur Formel I angegebenen Bedeutungen haben und $R^1$ die zur Formel I angegebene Bedeutung hat, jedoch kein Wasserstoffatom oder Kation bedeutet, umestert,

1 g) gegebenenfalls eine Verbindung der Formel I, worin A und $R^2$ die zur Formel I angegebenen Bedeutungen haben und $R^1$ einen Alkylrest bedeutet, zu einer Verbindung der Formel I verseift, worin $R^1$ Wasserstoff bedeutet,

1 h) gegebenenfalls eine Verbindung der Formel I, worin A und $R^2$ die zur Formel I angegebenen Bedeutungen haben und $R^1$ Wasserstoff bedeutet, mit Basen in eine Verbindung der Formel I überführt, worin $R^1$ ein physiologisch verträgliches Metall-, $NH_4$- oder Ammoniumion bedeutet, das sich von einem primären, sekundären oder tertiären Amin ableitet, oder

2 a) eine Verbindung der Formel VI

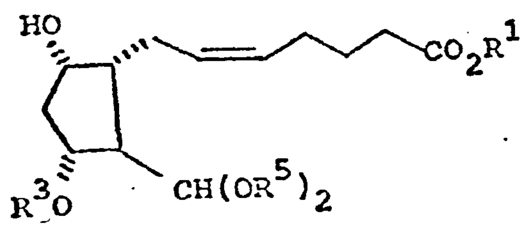

VI

worin $R^1$ die zur Formel I und $R^3$ die zur Formel II angegebenen Bedeutungen haben, und $R^5$ einen Alkylrest mit 1 bis 4 Kohlenstoffatomen bedeutet, mit einem elektrophilen Reagens umsetzt, wobei eine Verbindung der Formel VII

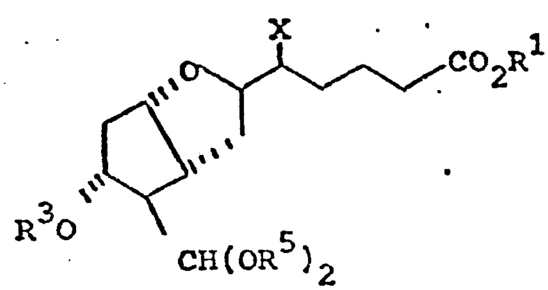

VII

gebildet wird, worin $R^1$ die zur Formel I, $R^3$ die zur Formel II, $R^5$ die zur Formel VI und X die zur Formel III angegebenen Bedeutungen haben,

2 b) aus einer Verbindung der Formel VII die Gruppe X reduktiv abspaltet, wobei eine Verbindung der Formel VIII

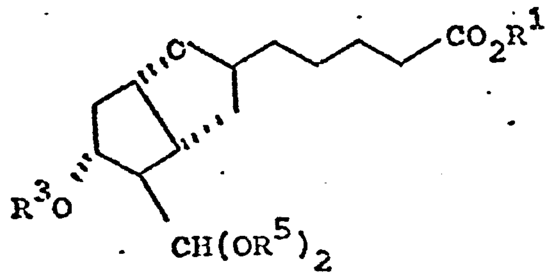

VIII

erhalten wird, worin $R^1$ die zur Formel I, $R^3$ die zur Formel II und $R^5$ die zur Formel VI angegebenen Bedeutungen haben,

2 b') gegebenenfalls in einer Verbindung der Formel VIII, worin $R^3$ Wasserstoff bedeutet, diesen durch eine Schutzgruppe ersetzt,

2 c) aus einer Verbindung der Formel VIII durch saure Hydrolyse die Acetalschutzgruppe entfernt, wobei eine Verbindung der Formel IX

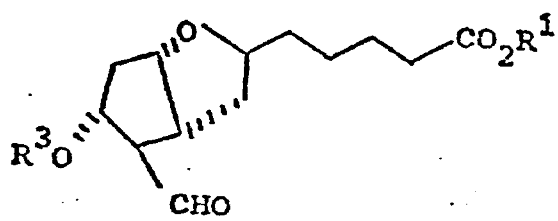

IX

entsteht, worin $R^1$ die zur Formel I und $R^3$ die zur Formel II angegebenen Bedeutungen haben,

2 d) einen Aldehyd der Formel IX mit einem Phosphonat der Formel X

$$(R^6O)_2\overset{O}{\overset{\|}{P}} - CH_2 - \overset{O}{\overset{\|}{C}} - R^2 \qquad X$$

worin $R^6$ einen Alkylrest mit 1 bis 4 Kohlenstoffatomen bedeutet und $R^2$ die zur Formel I angegebene Bedeutung hat, unter Basenkatalyse kondensiert, wobei eine Verbindung der Formel XI

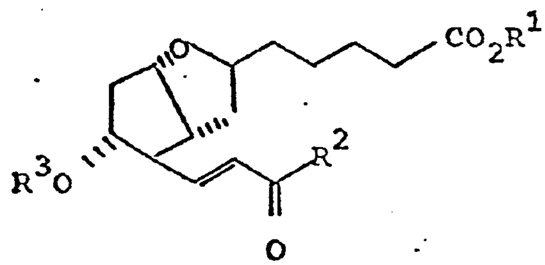

XI

entsteht, worin $R^1$ und $R^2$ die zur Formel I und $R^3$ die zur Formel II angegebene Bedeutung haben,

2 e) ein Keton der Formel XI zu einem Alkohol der Formel IV reduziert, worin $R^1$ und $R^2$ die zur Formel I, $R^3$ die zur Formel II angegebenen Bedeutungen haben und $R^4$ Wasserstoff bedeutet und gegebenenfalls weiter verfährt wie unter 1 c) bis 1 h) angegeben.

3. Verfahren zur Herstellung von Arzneimitteln, dadurch gekennzeichnet, daß man eine Verbindung der in Anspruch 1 genannten Formel I, gegebenenfalls mit üblichen pharmazeutischen Trägern und/oder Stabilisatoren, in eine therapeutisch geeignete Darreichungsform bringt.

4. Arzneimittel, gekennzeichnet durch einen Gehalt an einer Verbindung der in Anspruch 1 genannten Formel I.

5. Verwendung einer Verbindung der in Anspruch 1 genannten Formel I bei der Behandlung von Herzkreislauf-Krankheiten.

0001270

Europäisches Patentamt

# EUROPÄISCHER RECHERCHENBERICHT

Nummer der Anmeldung

EP 78 100 907.1

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| X | JOURNAL OF THE AMERICAN CHEMICAL SOCIETY, Vol. 99, No. 6, March 16, 1977. Columbus, Ohio. E.J. COREY et al "Synthesis of Vane's Prostaglandin X, 6,9α-Oxido-9α,15α-dihydroxyprosta-(Z)5, E(13)-dienoic Acid", Seite 2006 bis 2008 -- | 1,2 | C 07 D 307/93<br>A 61 K 31/34//<br>C 07 C 177/00 |
| PX | DE - A - 2 720 998 (WELLCOME) * Ansprüche 1, 13, 23 * -- | 1-5 | RECHERCHIERTE SACHGEBIETE (Int. Cl.²)<br>C 07 C 307/93<br>A 61 K 31/34 |
| PX | DE - A - 2 724 555 (UPJOHN) * Ansprüche 10 - 12 * -- | 1,2 | |
| - | DE - A - 2 702 553 (UPJOHN) * Anspruch 1 * -- | 2 | |
| - | JOURNAL OF THE CHEMICAL SOCIETY CHEMICAL COMMUNICATIONS, No. 10, May 18, 1977, London, K.C. NICOLAOU et al "Synthesis of (4E)-9-Deoxy-6,9α-epoxy-$\Delta^4$-$PGF_{1\alpha}$, a Prostacyclin (PGX) Isomer" Seite 331 bis 332 -- ./.. | 2 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| Berlin | 7-12-1978 | FROELICH |

EPA Form 1503.1 06.78

Europäisches Patentamt

**EUROPÄISCHER RECHERCHENBERICHT**

Nummer der Anmeldung

EP 78 100 907.1



| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| - | TETRAHEDRON LETTERS, No. 30, July 1977, OXFORD, I. TÖMÖSKÖZI et al "A simple Synthesis of $PGI_2$" Seite 26[illegible] bis 2628 | 2 | |
| | -- | | |
| PA | DE - A - 2 [illegible] 899 (WELLCOME) | | |
| | -- | | RECHERCHIERTE SACHGEBIETE (Int. Cl.²) |
| PA | FR - A - 2 [illegible] (PFIZER) | | |
| | -- | | |
| P | FR - A - 2 367 407 (WELLCOME) * Anspruch [illegible] * | 3,4,5 | |
| | ---- | | |